# EUROPEAN PATENT APPLICATION

(11) **EP 3 809 140 A1**
(43) Date of publication of application: **21.04.2021**
(21) Application number: 20203154.8
(22) Date of filing: 29.10.2015
(51) Int. Cl.: G01N 33/68

(54) **METHOD FOR THE ENRICHMENT OF CIRCULATING TUMOR DNA**

(30) Priority: 29.10.2014 GB 201419225; 30.10.2014 GB 201419299
(62) Divisional of application: 15790214.9
(71) Applicant: Belgian Volition SPRL, 5032 Isnes (BE)
(72) Inventor: MICALLEF, Jacob Vincent, BE-5032 Isnes (BE); HERZOG, Marielle, BE-5032 Isnes (BE); ECCLESTON, Mark Edward, BE-5032 Isnes (BE)
(74) Representative: Pond, Elizabeth Grace Catherine

(57) **Abstract**

The invention relates to the use of histone binding agents for detecting, isolating and/or purifying cell free nucleosomes of tumor origin or circulating tumor DNA from a biological sample. The invention also relates to methods and kits using said histone binding agents.

## Description

### FIELD OF THE INVENTION

The invention relates to a method for the purification or enrichment of circulating cell free nucleosomes of tumor origin and associated circulating tumor DNA from blood, serum or plasma.

### BACKGROUND OF THE INVENTION

Cellular DNA exists as a protein-nucleic acid complex called chromatin. The nucleosome is the basic unit of chromatin structure and consists of double stranded DNA (dsDNA) wound around a protein complex. The DNA is wound around consecutive nucleosomes in a structure often said to resemble "beads on a string" and this forms the basic structure of open or euchromatin. In compacted or heterochromatin this string is coiled and super coiled in a closed and complex structure.

Each nucleosome in chromatin consists of a protein complex of eight highly conserved core histones (comprising of a pair of each of the histones H2A, H2B, H3, and H4). Around this complex are wrapped approximately 146 base pairs (bp) of DNA. Cell free nucleosomes are reported to comprise 160-200bp DNA which may be due to the presence of further linker DNA. Another histone, H1 or H5, acts as a linker and is involved in chromatin compaction.

Normal cell turnover in adult humans involves the creation by cell division of some 10¹¹ cells daily and the death of a similar number, mainly by apoptosis. During the process of apoptosis chromatin is broken down into mononucleosomes and oligonucleosomes some of which may be found in the circulation. Under normal conditions the level of circulating nucleosomes found in healthy subjects is reported to be low. Elevated levels are found in subjects with a variety of conditions including many cancers, auto-immune diseases, inflammatory conditions, stroke and myocardial infarction (Holdenreider & Stieber, 2009).

DNA abnormalities are characteristic of all cancer diseases. The DNA of cancer cells differs from that of healthy cells in many ways including, but not limited to, point mutations, translocations, gene copy number, micro-satellite abnormalities, DNA strand integrity and nucleotide modifications (for example methylation of cytosine at position 5). These tumor associated alterations in DNA structure or sequence are investigated routinely in cancer cells or tissue removed at biopsy or surgery for clinical diagnostic, prognostic and treatment purposes. Tumor genetic and epigenetic characteristics vary between different tumor types and between different patients with the same tumor disease. Moreover these characteristics vary over time within the same cancer of the same patient with the progression of the disease and in the development of acquired resistance to drug or other therapies. Thus serial investigation of tumor DNA in cells removed at surgery or biopsy may help the clinician to monitor disease progression and detect any relapse or acquired treatment resistance at an early stage (possibly many months earlier than radiological detection) and allow potentially successful changes in treatment courses.

However, tissue DNA tests have limitations as invasive biopsy procedures cannot be performed repeatedly on patients for monitoring purposes. For some patients biopsy may not be used at all. Biopsy is expensive to perform, uncomfortable for the patient, poses patient risk, and may lead to surgical complications. Moreover, a tumor in a patient may consist of multiple tumoral clones located within different areas of the same tumor or within different metastases (in metastatic cancer) not all of which may be sampled on biopsy. A tissue biopsy DNA investigation therefore provides a snap-shot of the tumor, both in time and in space, amongst different tumor clones located within different areas of a tumor at a particular moment in time.

The blood of cancer patients contains circulating tumor DNA (ctDNA) which is thought to originate from the release of chromatin fragments or nucleosomes into the circulation from dying or dead cancer cells. Investigation of matched blood and tissue samples from cancer patients shows that cancer associated mutations, present in a patient's tumor (but not in his/her healthy cells) are also present in ctDNA in blood samples taken from the same patient (Newman *et al,* 2014). Similarly, DNA sequences that are differentially methylated (epigenetically altered by methylation of cytosine residues) in cancer cells can also be detected as methylated sequences in ctDNA in the circulation. In addition the proportion of cell-free circulating DNA (cfDNA) that is comprised of ctDNA is related to tumor burden so disease progression may be monitored both quantitatively by the proportion of ctDNA present and qualitatively by its genetic and/or epigenetic composition. Analysis of ctDNA can produce highly useful and clinically accurate data pertaining to DNA originating from all or many different clones within the tumor and which hence integrates the tumor clones spatially. Moreover, repeated sampling over time is a much more practical and economic option. Analysis of (ctDNA) has the potential to revolutionize the detection and monitoring of tumors, as well as the detection of relapse and acquired drug resistance at an early stage for selection of treatments for tumors through the investigation of tumor DNA without invasive tissue biopsy procedures. Such ctDNA tests may be used to investigate all types of cancer associated DNA abnormalities (e.g.; point mutations, nucleotide modification status, translocations, gene copy number, micro-satellite abnormalities and DNA strand integrity) and would have applicability for routine cancer screening, regular and more frequent monitoring and regular checking of optimal treatment regimens (Zhou *et al,* 2012).

Blood, plasma or serum may be used as a substrate for ctDNA assays and any DNA analysis method may be employed including, without limitation, DNA sequencing, epigenetic DNA sequencing analysis (e.g., for sequences containing 5-methylcytosine), PCR, BEAMing, NGS (targeted or whole genome), digital PCR, cold PCR (co-amplification at lower denaturation temperature-PCR), MAP (MIDI-Activated Pyrophosphorolysis), PARE (personalized analysis of rearranged ends) and Mass Spectrometry.

As DNA abnormalities are characteristic of all cancer diseases and ctDNA has been observed for all cancer diseases in which it has been investigated, ctDNA tests have applicability in all cancer diseases. Cancers investigated include, without limitation, cancer of the bladder, breast, colorectal, melanoma, ovary, prostate, lung liver, endometrial, ovarian, lymphoma, oral, leukaemias, head and neck, and osteosarcoma (Crowley *et al,* 2013; Zhou *et al,* 2012; Jung *et al,* 2010). The nature of ctDNA tests will now be illustrated by outlining three (non-limiting) example approaches.

The first example involves the detection of a cancer associated gene sequence mutation in ctDNA. Blood tests involving the detection of a single gene mutation in ctDNA generally have low clinical sensitivity. There are two reason for this. Firstly, although all cancers have mutations, the frequency of any particular mutation in a particular cancer disease is usually low. For example, although K-ras and p53 mutations are regarded as two of the more frequent cancer mutations and have been studied in a wide range of cancers including bladder, breast, colon, lung, liver, pancreas, endometrial and ovarian cancers, they were detected in 23%-64% and 17%-54% of cancer tissue samples respectively. Secondly, even if the cancer tissue of a patient does contain the mutation, the level or concentration of mutated ctDNA present in the blood of the patient may be low and difficult to detect. For example, K-ras and p53 mutations could be detected in the ctDNA of 0%-75% of K-ras and p53 tissue positive patients. The sum of these two effects meant that K-ras or p53 mutations were detected in the blood of less than 40% of cancer patients (Jung *et al,* 2010).

The second example involves the detection of multiple cancer associated gene sequence mutations in ctDNA. Although mutations of any particular gene such as K-ras or p53 may be present in only a minority of cancers, all cancers contain mutations so study of a sufficiently large panel of mutations should in principle, facilitate the detection of most or even all tumors. One way to increase the clinical sensitivity of such tests is therefore to test for a wide range of mutations in many genes. Newman *et al* have taken this approach for non-small cell lung cancer (NSCLC) and investigated 521 exons and 13 intron sequences from 139 recurrently mutated genes. The mutations studied encompassed multiple classes of cancer associated genetic alterations, including single nucleotide variation (SNV) and fusion genes. In this way the authors reported the detection of more than 95% of stage II-IV tumors and 50% of stage I tumors with 96% specificity in ctDNA blood tests (Newman *et al,* 2014).

The third example involves the detection of cancer associated epigenetic alterations to particular gene sequences in ctDNA. This approach can be applied to any DNA or nucleotide modification. A prime example of this approach is the detection of genes which are differentially methylated at cytosine residues in certain cancers. A large number of genes have been investigated for this purpose in a variety of cancers. A few of these are SEPTIN-9, APC, DAPK, GSTP1, MGMT, p16, RASSF1A, T1G1, BRCA1, ERa, PRB, TMS1, MLH1, HLTF, CDKN2A,SOCS1, SOCS2, PAX5, PGR, PTGS2 and RARβ2 investigated in bladder, breast, colorectal, melanoma, ovarian and prostate cancers. An illustrative example of this approach is the detection of methylated SEPTIN-9 in ctDNA for the detection of ColoRectal Cancer (CRC) which was reported to detect 68% of CRC cases with a clinical specificity of 89% (Grutzmann *et al,* 2008).

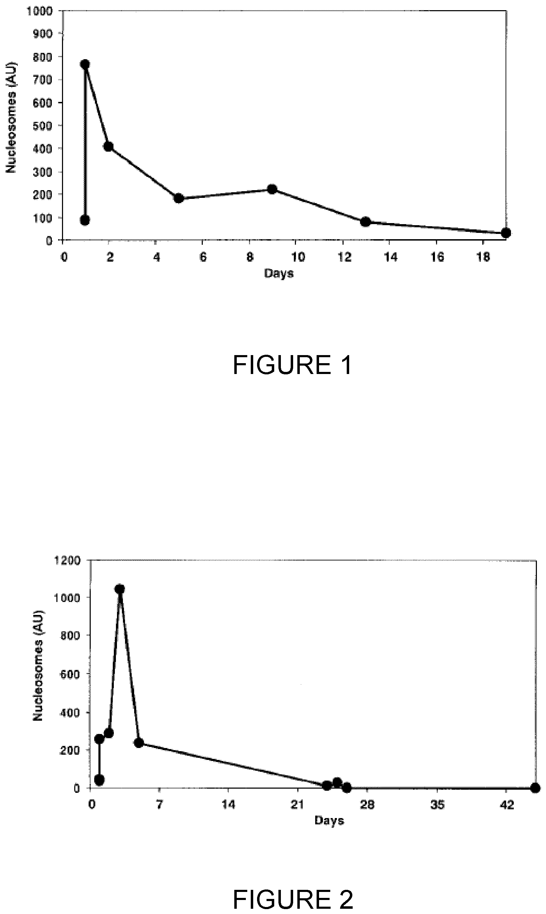

The tumor derived ctDNA fraction of cfDNA circulates as small DNA fragments with a median length of approximately 180 base pairs (bp) which is the size expected for DNA fragments circulating in the form of mono-nucleosomes (Newman *et al,* 2014). These 180bp DNA fragments are thought to comprise the nucleosomal DNA plus some linker DNA. Cancer patients are reported to have higher cfDNA levels than healthy subjects. Workers in the field have reported ranges of 0-100 ng/ml (mean 30 ng/ml) cfDNA for healthy subjects and 0-1000 ng/ml (mean 180 ng/ml) cfDNA for subjects with cancer (Schwarzenbach *et al,* 2011). Circulating cfDNA consists of DNA molecules of various sizes up to 20,000 base pairs in length (Zhou *et al,* 2012). In agreement with the hypothesis that ctDNA circulates predominantly as mono-nucleosomes, measured levels of cell free nucleosomes in the circulation are, like DNA levels, higher in cancer patients than in healthy subjects (Holdenrieder *et al,* 2001). However, raised levels of circulating nucleosomes *per se* are not used clinically as biomarkers of cancer as nucleosomes are a non-specific product of cell death and raised levels are observed for many conditions involving elevated cell death including acute trauma (Holdenrieder and Stieber, 2009). As a product of cell death, circulating nucleosome levels can rise markedly on treatment with cytotoxic drugs or radiotherapy. However, nucleosomes are also cleared from the circulation so levels may spike with treatment and then fall as shown in Figures 1 and 2 reproduced from Holdenrieder *et al,* 2001.

Although the level of circulating cell free nucleosomes *per se* has not been used in clinical practice as a blood based biomarker in cancer, the epigenetic composition of circulating cell free nucleosomes in terms of their histone modification, histone variant, DNA modification and adduct content have been investigated as blood based biomarkers in cancer (WO 2005/019826; WO 2013/030577; WO 2013/030579; WO 2013/084002).

The biological origin of cfDNA is not well understood. Fragmentation of chromatin to produce mononucleosomes and oligonucleosomes is a feature of apoptotic cell death. Necrotic cells are thought to produce larger DNA molecules of thousands of base pairs in length, but DNA fragmentation may also occur in some cases of necrosis. Further, common DNA repeat sequences (eg; ALU or LINE1 sequences) may be released as 200-400 base pair DNA fragments from cells undergoing non-apoptotic or necrotic cell death (Schwarzenbach *et al,* 2011). DNA fragments may also be secreted by cells as a form of inter-cellular communication. The origin of ctDNA is thought to be related to the death of cancer cells. DNA fragments may be released as nucleosomes from necrotic and/or apoptotic tumor cells. However, necrotic and apoptotic cells are usually phagocytosed by macrophages or other scavenger cells and DNA may be released by macrophages that have engulfed necrotic or apoptotic cells (Schwarzenbach *et al,* 2011).

There are a variety of methods available for extracting cfDNA from blood, serum or plasma and these have been compared for yield of extracted DNA and for their efficiency of extraction of DNA fragments of different lengths. Phenol-chloroform and sodium iodide extraction methods provide the highest yield and extract small DNA fragments of less than 200bp in length. Other methods tested (including commercially available methods) are reported to have lower DNA extraction yields and to fail to extract small DNA fragments of less than 200bp in length (Fong *et al,* 2009).

Extraction of cfDNA from blood, serum or plasma for analysis of ctDNA is usually performed using commercially available DNA extraction products. Such extraction methods claim high recoveries of circulating DNA (>50%) and some products (for example; the QIAamp Circulating Nucleic Acid Kit produced by Qiagen) are claimed to extract DNA fragments of small size. Typical sample volumes used are in the range 1-5mL of serum or plasma.

There are currently no ctDNA based tests in routine use for clinical oncology purposes due to a number of limitations. A major methodological limitation is a requirement for high quality DNA. Current ctDNA sampling methods produce poor quality ctDNA samples due to the nature of the sample. The main difficulty lies in the presence of large amounts of non-tumor cfDNA in the circulation which complicates any analysis of ctDNA. Estimates from different workers vary but the fraction of ctDNA present in the circulation can be too low to detect or above 50% of cfDNA. However, for most cancer patients the ctDNA fraction is a small part of cfDNA. For example, recent studies report that the ctDNA fraction increases with tumor size in pre-treatment lung cancer patients. The highest level found was 3.2% in a patient with a large tumor burden but most patients were found to have ctDNA fractions below 0.1% (Newman *et al,* 2014). This means that for many patient samples, a very low level of ctDNA must be analysed in the presence of a much higher level of non-tumor derived DNA. Moreover this DNA is from the same subject and hence of similar sequence and will interfere in any method for the quantification or analysis of ctDNA.

A similar problem occurs for the measurement of circulating cell free nucleosomes and/or the epigenetic composition of circulating nucleosomes as biomarkers for cancer because nucleosomes *per se* are a non-specific indicator of cell death and are released as part of the normal cell turnover process of the body as well as in conditions associated with elevated levels of cell death such as autoimmune diseases, stroke, sepsis, post trauma, burns, myocardial infarction, cerebral stroke, during graft rejection after organ transplantation and after severe exercise. Thus nucleosomes of tumor origin circulate together with other non-tumor nucleosomes of various cellular and tissue origins. These non-tumor nucleosomes will interfere in any method for the quantification or epigenetic analysis of nucleosomes of tumor origin.

There is therefore a great need for a method for the enrichment of circulating nucleosomes and ctDNA of tumor origin from blood, serum or plasma samples.

### SUMMARY OF THE INVENTION

According to one aspect of the invention, there is provided the use of a histone H3.1 and/or H3.2 and/or H3t binding agent for detecting, isolating and/or purifying cell free nucleosomes of tumor origin or circulating tumor DNA (ctDNA) from a biological sample.

According to a further aspect of the invention, there is provided a method for isolating circulating cell free nucleosomes of tumor origin from a biological sample by affinity purification wherein said method comprises the steps of:
(i) contacting the sample with a histone H3.1 and/or H3.2 and/or H3t binding agent;
(ii) isolating bound nucleosomes from the sample; and
(iii) analysing the isolated nucleosomes and/or associated DNA.

According to a further aspect of the invention, there is provided a method for isolating purified circulating tumor DNA (ctDNA) from a biological sample, wherein said method comprises the steps of:
(i) isolating circulating cell free nucleosomes containing histone H3.1 and/or H3.2 and/or H3t;
(ii) extracting DNA from the nucleosome sample produced in step (i); and
(iii) analysing the extracted DNA.

According to a further aspect of the invention, there is provided an immunoassay method for detecting an epigenetic epitope of tumor derived circulating nucleosomes in a biological sample, wherein said method comprises the steps of:
(i) contacting the sample with a histone H3.1 and/or H3.2 and/or H3t binding agent;
(ii) contacting the nucleosomes or sample with a second binding agent which binds to said epitope;
(iii) detecting and/or quantifying the binding of said second binding agent to said epitope; and
(iv) using the presence or degree of such binding as a measure of the presence of the particular epitope of tumor derived nucleosomes in the sample.

According to a further aspect of the invention, there is provided an immunoassay method for detecting an epigenetic epitope of tumor derived circulating nucleosomes in a biological sample wherein said method comprises the steps of:
(i) contacting the sample with a first binding agent which binds to said epitope;
(ii) contacting the nucleosomes or sample with a histone H3.1 and/or H3.2 and/or H3t binding agent;
(iii) detecting and/or quantifying the binding of said histone H3.1 and/or H3.2 and/or H3t binding agent to nucleosomes in the sample; and
(iv) using the presence or degree of such binding as a measure of the presence of the particular epitope of tumor derived nucleosomes in the sample.

According to a further aspect of the invention, there is provided a method of diagnosing cancer which comprises the step of detecting circulating cell free nucleosome associated histone variant H3.1 and/or H3.2 and/or H3t in a biological sample obtained from a human or animal subject.

### BRIEF DESCRIPTION OF THE FIGURES

**Figure 1****:** Circulating levels of cell-free nucleosomes *per se* in a patient with head and neck cancer, treated with 16 daily doses of radiotherapy. Levels were measured with a commercial nucleosome ELISA employing an immobilised antibody directed to bind to a core nucleosome epitope (copied from Holdenrieder *et al;* 2001)
**Figure 2****:** Circulating levels of cell-free nucleosomes *per se* in a patient with small-cell lung cancer, treated with cytotoxic chemotherapy on day 1. Levels were measured over more than 42 days with a commercial nucleosome ELISA employing an immobilised antibody directed to bind to a core nucleosome epitope (copied from Holdenrieder *et al,* 2001)

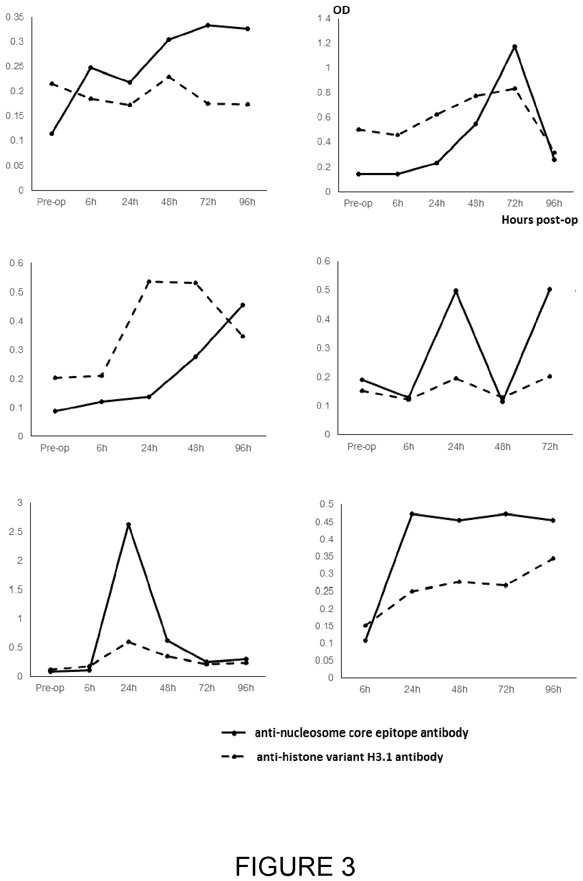

**Figure 3****:** Circulating levels of cell-free nucleosomes *per se* and circulating levels of cell-free nucleosomes containing histone variant H3.1 and/or H3.2 and/or H3t in 6 patients with CRC pre-surgery and at 6, 24, 48, 72 and 96 hours post-surgery. Levels of cell-free nucleosomes *per se* were measured with a commercial nucleosome ELISA employing an immobilised antibody directed to bind to a core nucleosome epitope. Levels of circulating cell-free nucleosomes containing histone variants were measured with a similar assay but employing an immobilized antibody directed to bind to histone variants H3.1, H3.2 or H3t.

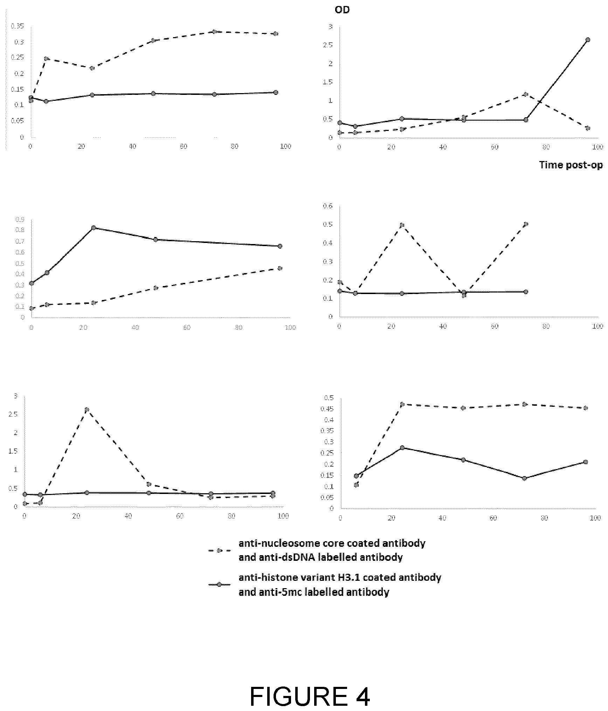

**Figure 4****:** Circulating levels of cell-free nucleosomes *per se* and circulating levels of cell-free nucleosomes containing histone variant H3.1, H3.2 or H3t and 5-methylcytosine (5mc) in 6 patients with CRC pre-surgery and at 6, 24, 48, 72 and 96 hours post-surgery. Levels of cell-free nucleosomes *per se* were measured with a commercial nucleosome ELISA employing an immobilised antibody directed to bind to a core nucleosome epitope. Levels of circulating cell-free nucleosomes containing histone H3 variants and 5mc were measured with an assay employing an immobilized antibody directed to bind to the histone variant H3.1, H3.2 or H3t and a labelled antibody directed to bind to 5mc.

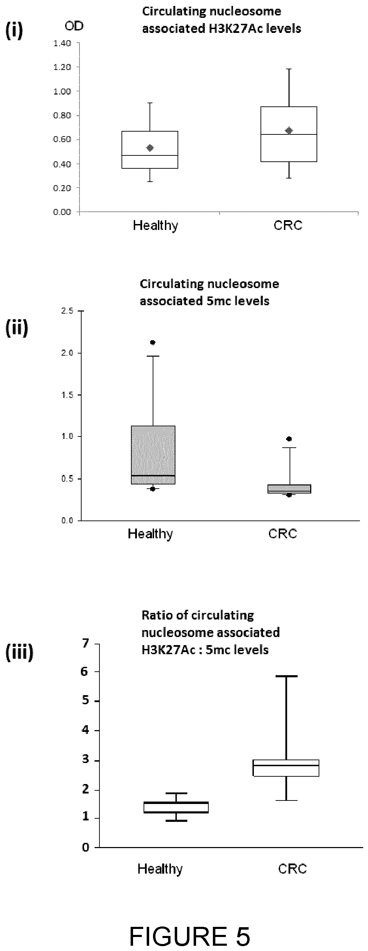

**Figure 5****:** Circulating levels of cell-free nucleosomes containing (i) both histone variant H3.1, H3.2 or H3t and histone modification H3K27Ac or (ii) both histone variant H3.1, H3.2 or H3t and 5-methylcytosine (5mc) in healthy patients and patients with CRC. Expression of the levels of circulating cell-free nucleosomes containing the histone H3 variant as well as histone modification H3K27Ac as a ratio to those containing 5mc, leads to improved discrimination between blood samples taken from healthy subjects and subjects with CRC (iii).

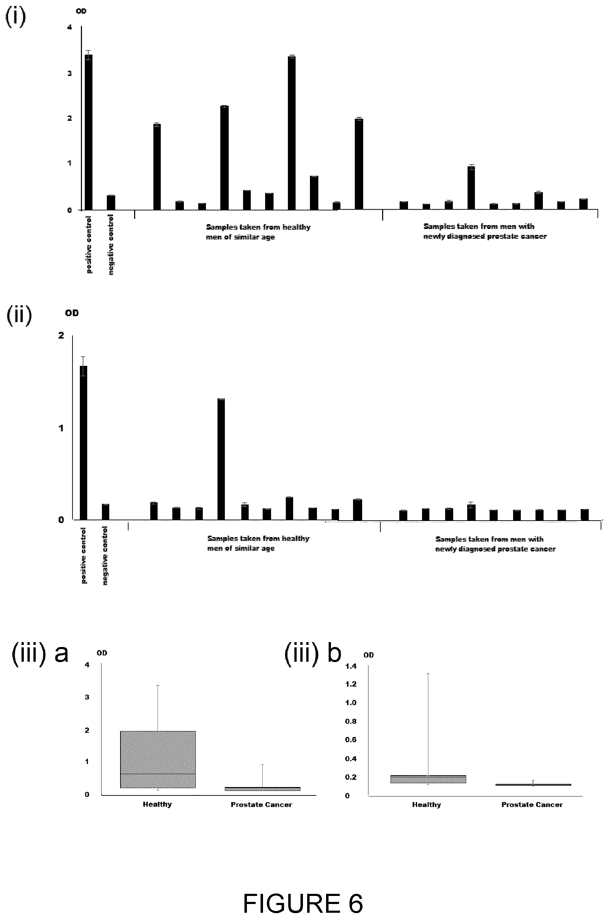

**Figure 6****:** Circulating levels of cell-free nucleosomes containing (i) both histone variant H3.1, H3.2 or H3t and 5-methylcytosine or (ii) nucleosomes *per se* containing 5-methylcytosine (5mc) in newly diagnosed patients with prostate cancer and healthy control subjects of similar age. The data are also displayed as box-plots for cell-free nucleosomes containing (iii) a, both histone variant H3.1, H3.2 or H3t and 5-methylcytosine or (iii) b, nucleosomes *per se* containing 5-methylcytosine (5mc) patients.

### DETAILED DESCRIPTION OF THE INVENTION

The structure of nucleosomes in terms of their epigenetic signal composition may vary in cancer cells. The use of antibodies or other binders directed to bind to epigenetic signals that are more common in cancer cells than in other cells may be selective for the binding of cell free nucleosomes of tumor origin in a biological sample taken from a subject which contains cell free nucleosomes with a mixture of cellular origins.

According to a first aspect of the invention, there is provided the use of a histone H3.1 and/or H3.2 and/or H3t binding agent for detecting, isolating and/or purifying circulating tumor DNA (ctDNA) from a biological sample.

In one embodiment, cell free nucleosomes of tumor origin are isolated and/or purified.

The ctDNA in patient samples is often present in very low or undetectable concentrations and furthermore comprises only a small proportion of the cfDNA present. The clinical performance and utility of cancer tests based on ctDNA analysis would be improved if better quality samples were available. Similarly circulating cell free nucleosomes of tumor origin circulate as part of a mixture of nucleosomes with a variety of origins and comprise only a proportion of the cell free nucleosomes present. Surprisingly we now show that enrichment or isolation of nucleosomes of tumor origin, together with the nucleosome associated ctDNA fraction of cfDNA, from blood, serum or plasma samples may be performed using an affinity purification isolation method.

Holdenrieder has reported that elevated circulating cell-free nucleosome levels are characteristic of malignant and benign tumor diseases (Holdenrieder *et al,* 2001). However, this is not useful for the detection of cancer as circulating nucleosome levels are a non-specific marker for cell death and are elevated in a wide variety of conditions including autoimmune diseases, stroke, sepsis, post trauma, burns, myocardial infarction, cerebral stroke, during graft rejection after organ transplantation and after severe exercise (Holdenrider & Stieber, 2009). Holdenrieder measured circulating nucleosomes using an ELISA technique in which the first immobilized antibody employed was directed to bind to a common nucleosome core epitope and the second labelled antibody was directed to bind double-stranded DNA. This ELISA method is designed to detect all nucleosomes, or nucleosomes *per se*, complete with associated dsDNA regardless of epigenetic structure.

Circulating nucleosome levels can spike markedly 2-5 days after a sudden increase in cell death resulting from any number of disparate causes including trauma, stroke or treatment with cytotoxic drugs or radiotherapy. Levels then fall over a period of 2-3 days as shown in Figures 1 and 2 (reproduced from Holdenrieder *et al,* 2001). This effect is due to induction of cell death followed by clearance from the circulation (Holdenrider & Stieber, 2009).

We have performed similar experiments using the same commercially available nucleosome ELISA, employing the same immobilized antibody directed to bind to a common nucleosome core epitope and the same labelled antibody directed to bind to double-stranded DNA, used by Holdenrieder *et al,* to detect all nucleosomes regardless of epigenetic status. In these experiments we measured circulating cell-free nucleosome levels in 6 patients with CRC pre-surgery and at 6, 24, 48, 72 and 96 hours post-surgery. In agreement with the findings of Holdenrieder, a post-surgery rise in the level of nucleosomes was observed in every case as shown in Figure 3. The detailed timing of the rise in nucleosome levels, as well as the timing of any subsequent fall, varied between patients.

It is clear that nucleosomes released into the circulation of patients with no tumor disease (including for example due to surgical trauma) cannot have a tumor origin. These nucleosomes will contribute to cfDNA but will not contain ctDNA. It will also be clear to those skilled in the art, that nucleosomes released post-surgery in our own experiments may have a tumor origin but may also have a non-tumor origin due to the trauma of surgery. The fraction of such cfDNA that has a tumor origin (i.e.; the ctDNA fraction) may be determined as the allelic fraction of the cfDNA which contains tumor associated mutations. We have developed a method whereby circulating nucleosomes of tumor origin containing ctDNA may be enriched or isolated from other nucleosomes and cfDNA.

We re-assayed the samples taken from the 6 CRC patients pre-surgery and post-surgery using a second ELISA method. This second ELISA method employed the same labelled antibody directed to bind to double-stranded DNA as the commercially available method used by Holdenrieder, but employed an immobilized antibody directed to bind to histone variant H3.1, H3.2 or H3t. The results show that the response in the level of nucleosomes containing H3.1, H3.2 or H3t to surgery is dissimilar to that of the response observed for nucleosomes *per se* using the ELISA employing an antibody directed to bind to a common nucleosome core epitope. The level of circulating nucleosomes containing these H3 variants has different response characteristics to the level of (total) circulating nucleosomes *per se,* and is less affected by surgery (Figure 3).

A variety of epigenetically modified nucleotides have been described in the literature and epigenetic modification patterns in DNA and/or DNA nucleotide residues are known to be altered in cancer. The best described of these includes methylation of cytosine at position 5. DNA containing 5-methylcytosine is often referred to as methylated DNA. We developed and performed a third ELISA method on the same 6 CRC patients. This ELISA was a method for the measurement of nucleosomes containing DNA incorporating 5-methylcytosine residues as an example of an assay for DNA containing an epigenetically modified nucleotide as described in WO 2013/030577 but using an immobilized anti-H3.1, H3.2 or H3t antibody as capture antibody. This third ELISA method thus detected cell free nucleosomes containing both histone variant H3.1, H3.2, or H3t as well as 5-methylcytosine residues and was thus similar to the second ELISA method described above but used a different labelled antibody (directed to bind to 5-methylcytosine rather than dsDNA). The results for the 6 CRC patients show that the level of H3 variant nucleosome associated 5-methylcytosine is low and less affected by surgery than the level of (total) circulating nucleosomes *per se* (Figure 4).

In addition we have used this assay to measure concurrent histone variant H3.1, H3.2, or H3t with 5-methylcytosine methylated DNA levels in circulating nucleosomes in healthy subjects and subjects with CRC. The level of methylated DNA in cancer subjects was lower than in healthy subjects. This finding agrees with the published literature finding that DNA is globally hypo-methylated in cancer cells. The methylation of DNA in cancer cells is estimated to be reduced by approximately 50% compared to the DNA of healthy cells (Guerrero-Preston *et al,* 2007; Soares *et al,* 1999). However, the cancer associated increase in the level of circulating nucleosomes is reported to be 970% on average (Holdenrieder *et al,* 2001) and the increase in cfDNA to be about 600% (Schwarzenbach *et al,* 2011). The large increase in total cfDNA might be expected to lead to an overall rise in the absolute levels of circulating nucleosome associated methylated DNA, despite the (smaller) fall in the proportion of circulating cell-free nucleosomes containing methylated DNA. However, the results of the present assay employing a capture antibody to histone variant H3.1, H3.2 or H3t show a decrease in the absolute level of circulating nucleosomes containing methylated DNA (and histone H3 variant) in cancer patients, indicating that a large part of the cancer associated observed rise in (total) circulating nucleosomes *per se* assay is not of tumor cell origin. Moreover, we have shown this H3 variant nucleosome associated 5-methylcytosine assay to be effective for the detection of cancer in a blood test indicating that all or a large proportion of the cell free nucleosomes identified by it are of tumor origin. A box plot showing results of this assay for cancer patients and healthy patients is shown in Figure 5.

We have also developed and performed a fourth ELISA method for the measurement of circulating cell-free nucleosomes containing histone variant H3.1, H3.2 or H3t and the histone modification H3K27Ac (acetylated lysine 27 of histone H3). This is similar to the assays for nucleosomes containing an epigenetically modified histone as described in the literature (WO 2005/019826) but employed an immobilized anti-H3.1, H3.2 or H3t antibody as capture antibody. This fourth ELISA was identical to the third ELISA method described above except that it employed a different labelled antibody directed to bind to H3K27Ac (rather than 5-methylcytosine). We have similarly used this fourth assay to measure concurrent H3.1, H3.2 or H3t and H3K27Ac levels in the same cell free circulating nucleosomes in healthy subjects and subjects with CRC. The level of H3K27Ac in cancer subjects was higher than in healthy subjects. This finding agrees with the published literature finding that acetylation of H3K27 is elevated in the chromatin of cancer cells (Karczarski *et al,* 2014). Moreover, we have shown this assay to be effective for the detection of cancer in a blood test indicating that all or a large proportion of the cell free nucleosomes identified by it are of tumor origin. A box plot showing results of this assay for cancer patients and healthy patients is shown in Figure 5. The assay results differentiate between blood samples taken from healthy subjects and subjects with cancer. When the results of the third and fourth ELISA assays described here for epigenetically modified circulating cell free nucleosomes containing histone H3 variants as well as 5-methylcytosine (5mc) or H3K27Ac respectively, the discrimination between subjects with cancer and healthy subjects is enhanced further. These results show that assays for circulating cell free nucleosomes containing both the histone modification H3K27Ac as well as histone variant H3.1, H3.2 or H3t are useful in clinical oncology including, without limitation, for the detection of cancer, as well as for prognostic prediction, therapy selection, patient monitoring and relapse monitoring/detection. Assays for nucleosome associated H3K27Ac may be performed in isolation or as part of an assay panel comprising epigenetic and/or other tests.

In order to further confirm that the circulating nucleosome fraction containing the histone variant H3.1, H3.2 or H3t is enriched for nucleosomes of tumor origin and is useful for oncology blood tests, we performed an ELISA method using an immobilized anti-H3.1, H3.2 or H3t antibody as capture antibody and a labelled anti-5-methylcytosine antibody (ie; the third ELISA method described above) for nucleosomes containing both the histone variant H3.1, H3.2 or H3t as well as DNA incorporating 5-methylcytosine residues, on samples taken from 9 men newly diagnosed with prostate cancer and 10 healthy men of similar age. The men with prostate cancer were found to have lower circulating nucleosome associated 5-methylcytosine levels than healthy men and this assay may thus be used, either alone or as part of a diagnostic panel, as a method to detect prostate cancer. This ELISA was then repeated but using an immobilized antibody directed to bind to a common nucleosome core epitope (a fifth ELISA design). This fifth assay showed less discrimination for prostate cancer. The results are shown in Figure 6.

We conclude that circulating cell-free nucleosomes containing the histone variant H3.1, H3.2 or H3t may also contain other epigenetic signals. We also conclude that the levels of circulating cell-free nucleosomes containing both a histone H3 variant and another particular epigenetic signal may be different in cancer and healthy subjects and that this may happen in concordance with the levels observed in cancer cells. We further conclude that this remains true even though the level of the particular epigenetic signal may be elevated or depressed in the chromatin of cancer cells. The fact that otherwise identical ELISA methods, which capture exactly the same circulating nucleosome fraction and differ only in the epigenetic signaling structure targeted by the labelled antibody, can produce results that rise or fall (for the same circulating nucleosome fraction containing histone H3 variants) in concordance with the expression of the same epigenetic signals found in the chromatin of cancer cells indicates a tumor origin for this nucleosome fraction.

We further conclude that circulating nucleosomes containing a histone H3 variant also have dissimilar cellular release characteristics to other circulating nucleosomes. Such nucleosomes have different origins and are less a result of trauma induced cell death. As nucleosomes containing H3.1, H3.2 or H3t are not produced to a large extent by trauma induced cell death and can be used as a biomarker for cancer, it is clear that they are characteristic of cancer and have a predominantly tumor origin. Separation or isolation of nucleosomes containing histone H3.1, H3.2 or H3t produces an isolation or enrichment of circulating nucleosomes of tumor origin containing ctDNA from a bodily fluid sample such as blood, serum or plasma.

It will be clear to those skilled in the art that the level of nucleosomes containing a histone H3 variant as a proportion of nucleosomes present may be used as a measure of the proportion of cfDNA that comprises ctDNA in a sample. Such a measure is similar to allelic frequency measures of cancer associated mutations in ctDNA and may be used as a measure tumor burden and response to therapy.

Other epigenetic marks characteristic of cancer may similarly be useful in methods of the invention provided that they occur more frequently in circulating nucleosomes of tumor origin, or ctDNA, than other circulating nucleosomes, or cfDNA. The epigenetic signal need not be unique to nucleosomes or DNA of tumor origin, but the increased frequency should be sufficient to enrich nucleosomes and DNA of tumor origin. Such epigenetic marks may include histone variants (or isoforms), histone modifications, DNA modifications and nucleosome adducts.

In a first embodiment of the invention a wholly or partially purified tumor nucleosome preparation is isolated from a sample of a biological fluid. The purification method involves the affinity isolation of cell free nucleosomes containing a histone or DNA epigenetic signal epitope characteristic of cancer employing a binder that binds to the said epigenetic epitope. The tumor nucleosome preparation and/or its associated ctDNA may then be analysed. In a preferred embodiment tumor nucleosome and ctDNA isolation is performed by an immunological affinity purification method employing a binder to histone H3.1 or H3.2 or H3t. It will be clear to persons skilled in the art that any binding agent capable of specific binding to a histone H3 variant (or other appropriate nucleosome epitope characteristic of cancer) may be used for affinity purification methods of the invention. Such binding agents may include, without limitation, antibodies, aptamers or binding proteins (e.g.; nucleosome binding proteins).

Antibodies may be raised by a variety of methods known in the art including immunization and library methods such as phage display. The immune response may be induced against, or the library may be selected for binding to, the moiety or antigen of interest. Antibodies directed to bind to a histone H3 variant may be raised against a variety of such moieties including the whole histone H3 isoform protein amino acid sequence and may optionally contain post-translational histone modifications. The protein may be purified from living cells or produced synthetically. Alternatively a peptide sequence representing a part of the H3 isoform amino acid sequence may be used and this may also optionally contain post-translational histone modifications. Nucleosomes or other chromatin fractions containing histone H3 isoforms may also be used.

In the present investigations an antibody which binds to the peptide sequence A T K A A R K SAP A T G G V K K P H was employed. This amino acid sequence occurs in the sequence for histone variants H3.1, H3.2 and H3t but not in other histone H3 variants. It will be clear to those skilled in the art that binding agents directed to bind to any or all of histone variants H3.1 and/or H3.2 and/or H3t and/or H3.2 and/or H3t may be employed in methods of the invention. We have primarily referred herein to histone H3.1 but this notation is intended to include histone H3.1 and/or H3.2 and/or H3t throughout.

According to a further aspect of the invention, there is provided a method for isolating circulating cell free nucleosomes of tumor origin from a biological sample by affinity purification wherein said method comprises the steps of:
(i) contacting the sample with a histone H3.1 and/or H3.2 and/or H3t binding agent;
(ii) isolating bound nucleosomes from the sample; and
(iii) analysing the isolated nucleosomes and/or associated DNA.

The analysis of the isolated nucleosomes of tumor origin may involve any suitable method of analysis of which many are known in the art. These methods include without limitation analysis by ELISA using a second antibody or other binder to a common nucleosome epitope such as dsDNA or to an epigenetic structure of interest including a histone modification, histone variant, DNA modification or another molecule adducted to a nucleosome. These methods include herein by reference all the methods described in WO 2005/019826, WO 2013/030577, WO 2013/030579 and WO 2013/084002 wherein a histone H3 variant binder is employed in place of a general anti-nucleosome epitope binder so that circulating nucleosomes of tumor origin (rather than nucleosomes *per se*) are analysed for the epigenetic composition. These methods also include multiplex methods for the analysis of multiple epitopes present in circulating nucleosomes of tumor origin.

According to one embodiment of this aspect there is provided an immunoassay for the analysis of a particular epigenetic epitope of tumor derived circulating nucleosomes in terms of any particular nucleosome associated modified histone, histone variant, modified nucleotide or in terms of the presence of any another molecule adducted to a nucleosome which comprises the steps of:
(i) contacting the sample with a histone H3.1 and/or H3.2 and/or H3t binding agent;
(ii) contacting the nucleosomes or sample with a second binding agent which binds to said epitope;
(iii) detecting and/or quantifying the binding of said second binding agent to said epitope; and
(iv) using the presence or degree of such binding as a measure of the presence of the particular epitope of tumor derived nucleosomes in the sample.

According to an alternative embodiment there is provided a method for detecting and measuring cell free nucleosomes containing a particular epigenetic epitope, or composition of tumor derived circulating nucleosomes, in terms of any particular nucleosome associated modified histone, histone variant, modified nucleotide or in terms of the presence of another molecule adducted to a nucleosome which comprises the steps of:
(i) contacting the sample with a first binding agent which binds to said epitope;
(ii) contacting the nucleosomes or sample with a histone H3.1 and/or H3.2 and/or H3t binding agent;
(iii) detecting and/or quantifying the binding of said histone H3.1 and/or H3.2 and/or H3t binding agent to nucleosomes in the sample; and
(iv) using the presence or degree of such binding as a measure of the presence of the particular epitope of tumor derived nucleosomes in the sample.

The analysis of nucleosomes of tumor origin isolated by a method of the invention may also involve any proteomics method known in the art including without limitation electrophoresis methods, chromatographic methods and any method involving mass spectrometry including methods involving chromatography and mass spectrometry and/or stable isotope labelled mass spectrometry and/or methods involving protein digestion to produce peptides for identification and/or quantification by mass spectrometry or any combinatorial mass spectrometry method with any other method. In a preferred embodiment of the invention a circulating nucleosome preparation enriched for nucleosomes of tumor origin is prepared by affinity purification of circulating nucleosomes in a blood, serum or plasma sample taken from a cancer patient and the epigenetic composition of the nucleosome preparation is investigated by a method involving mass spectrometry. In one embodiment the method comprises the steps of:
(i) contacting the sample with a histone variant H3.1 and/or H3.2 and/or H3t binding agent;
(ii) isolating bound nucleosomes from the sample; and
(iii) analyzing the nucleosomes isolated in step (ii) using a method comprising mass spectrometry.

In one embodiment, isolation of nucleosomes containing ctDNA is performed by an immunological affinity purification method comprising the steps of:
(i) contacting the sample with binding agent directed to bind to an epigenetic epitope more commonly occurring in tumor derived than non-tumor derived nucleosomes;
(ii) isolating bound nucleosomes from the sample; and
(iii) optionally extracting DNA from the nucleosomes isolated in step (ii).

In a further embodiment, the binding agent directed to bind to an epigenetic epitope characteristic of tumor derived nucleosomes is directed to bind to histone variant H3.1 and/or H3.2 and/or H3t in an immunological affinity purification method comprising the steps of:
(i) contacting the sample with a histone variant H3.1 and/or H3.2 and/or H3t binding agent;
(ii) isolating bound nucleosomes from the sample; and
(iii) optionally extracting DNA from the nucleosomes isolated in step (ii).

Investigation of the purified or isolated ctDNA may involve analysis of any or all types of cancer associated DNA abnormalities including, without limitation, epigenetic analysis including the methylation of DNA sequences, point mutations, translocations, gene copy number, micro-satellite abnormalities and DNA strand integrity. Further any DNA analysis method may be employed including, without limitation, DNA sequencing, methylated DNA sequencing analysis, PCR, BEAMing, NGS (targeted or whole genome), digital PCR, cold PCR (co-amplification at lower denaturation temperature-PCR), MAP (MIDI-Activated Pyrophosphorolysis), PARE (personalized analysis of rearranged ends) and Mass Spectrometry.

In one embodiment, the biological sample comprises a blood, serum or plasma sample.

According to a further aspect of the invention, there is provided a method of diagnosing cancer which comprises the step of detecting circulating cell free nucleosome associated histone variant H3.1 and/or H3.2 and/or H3t in a biological sample obtained from a human or animal subject.

In one embodiment, the method of diagnosis additionally comprises detecting one or more histone modification, modified nucleotide, histone variant or isoform or nucleosome adduct.

In a further embodiment, the histone modification comprises H3K27Ac and/or 5-methylcytosine.

According to a further aspect of the invention, there is provided the use of a kit comprising a histone H3.1 and/or H3.2 and/or H3t binding agent in any of the methods described herein.

The invention will now be illustrated with reference to the following non-limiting examples.

### EXAMPLE 1

An antibody directed to bind specifically to histone isoform H3.1, H3.2 or H3t is biotinylated and immobilized on streptavidin coated magnetic beads (Dynal) by the recommended method of the manufacturer. The beads are washed several times with loading buffer using a magnetic separation system. Serum or plasma taken from a cancer patient is diluted in loading buffer and added to the beads. Nucleosomes containing histone H3 variant are adsorbed to the beads. Other serum/plasma components remain in solution and are removed by means of magnetic separation. The beads are washed with buffer. The nucleosomes containing histone H3 variant are now isolated on the beads. ctDNA associated with the isolated nucleosomes is extracted by the phenol/chloroform method or other standard extraction methods. The extracted DNA may be analysed for genetic or epigenetic features of cancer.

### EXAMPLE 2

An antibody directed to bind specifically to histone isoform H3.1, H3.2 or H3t is biotinylated and immobilized on streptavidin coated magnetic beads (Dynal) by the recommended method of the manufacturer. The beads are washed several times with loading buffer using a magnetic separation system. Serum or plasma taken from a cancer patient is diluted in loading buffer and added to the beads. Nucleosomes containing histone H3 variant are adsorbed to the beads. Other serum/plasma components remain in solution and are removed by magnetic separation. The beads are washed with buffer. The nucleosomes containing histone H3 variant are now isolated on the beads. The isolated nucleosomes are removed from the magnetic beads using an elution buffer and the nucleosomes are analysed by proteomics methods including Mass Spectroscopy.

### EXAMPLE 3

Serum samples taken from 6 patients with CRC pre-surgery and at 6, 24, 48, 72 and 96 hours post-surgery were assayed for circulating cell-free nucleosome levels using a commercial (total) nucleosome ELISA produced by Roche employing a common anti-nucleosome core epitope and a labelled anti-dsDNA antibody. The samples were then re-assayed but using an anti-histone variant H3.1, H3.2 or H3t capture antibody. The measured (total) nucleosome levels increased following the trauma of surgery using the commercial ELISA but the response to surgery was altered and muted for nucleosomes containing histone H3 variant measured using the assay employing anti-histone H3.1, H3.2 or H3t antibody. The results are shown in Figure 3.

### EXAMPLE 4

Serum samples taken from patients with CRC and healthy control subjects were assayed using an ELISA employing an anti-histone variant H3.1, H3.2 or H3t capture antibody and either a labelled anti-histone modification H3K27Ac antibody or a labelled anti-5-methylcytosine antibody. When the labelled anti-histone modification H3K27Ac antibody was used the results showed that a higher level of nucleosome associated H3K27Ac was present in the circulation of the cancer patients than in the healthy controls. Conversely, when the labelled anti-5-methylcytosine antibody was used the results showed that a lower level of nucleosome associated 5-methylcytosine was present in the circulation of the cancer patients than in the healthy controls (Figure 5). Both of these findings are consistent with the epigenetic alterations reported for the chromatin of cancer cells and tissue (decreased global DNA methylation and increased global H3K27 acetylation). Moreover, when the results are of the two epigenetically modified nucleosome results are expressed as a ratio the combined data differentiate healthy from CRC subjects with high accuracy as shown in Figure 5. These results indicate that these ELISA methods can be used to detect cancer and that the nucleosomes bound to the solid phase anti-histone H3 variant antibody are predominantly of tumor origin.

### EXAMPLE 5

Serum samples taken from 9 men newly diagnosed with prostate cancer and 10 healthy men of similar age were assayed with an ELISA method using an immobilized anti-H3.1, H3.2 or H3t antibody as capture antibody and a labelled anti-5-methylcytosine antibody for nucleosomes containing DNA incorporating 5-methylcytosine residues. The men with prostate cancer were found to have lower circulating nucleosome associated 5-methylcytosine levels than healthy men and this assay may thus be used, either alone or as part of a diagnostic panel, as a method to detect prostate cancer. This ELISA was then repeated but using an immobilized antibody directed to bind to a common nucleosome core epitope. This assay showed less discrimination for prostate cancer. The results are shown in Figure 6.

### REFERENCES

Crowley et al, Nature Reviews Clinical Oncology, 10, 472-484, 2013.
Fong et al, Clinical Chemistry 55(3), 587-589, 2009.Grutzmann et al, PLoS ONE 3(11): e3759. doi:10.1371/journal.pone.0003759, 2008.
Guerrero-Preston et al, Epigenetics 2(4), 223-226, 2007.
Holdenrieder et al, Int J Cancer 95, 114-120, 2001.
Holdenrieder and Stieber, Critical Reviews in Clinical Laboratory Sciences; 46(1): 1-24, 2009.
Jung et al, Clinica Chimica Acta, 411, 1611-1624, 2010.
Karczarski et al, Clinical Proteomics, 11:24, 2014.
Newman et al, Nature Medicine 20(5), 548-554, 2014.
Schwarzenbach et al, Nature Reviews Cancer, 11(6), 426-437, 2011.
Soares et al, Cancer 85(1), 112-118, 1999.
Zhou et al, Seminars in Oncology, 39(4), 440-448, 2012.

### EMBODIMENTS

1. Use of a histone H3.1 and/or H3.2 and/or H3t binding agent for detecting, isolating and/or purifying cell free nucleosomes of tumor origin or circulating tumor DNA (ctDNA) from a biological sample.
2. A method for isolating circulating cell free nucleosomes of tumor origin from a biological sample by affinity purification wherein said method comprises the steps of:
   (i) contacting the sample with a histone H3.1 and/or H3.2 and/or H3t binding agent;
   (ii) isolating bound nucleosomes from the sample; and
   (iii) analysing the isolated nucleosomes and/or associated DNA.
3. The method according to embodiment 2, wherein the step of analysing the isolated nucleosomes and/or associated DNA comprises an immunoassay method.
4. The method according to embodiment 2, wherein the step of analysing the isolated nucleosomes and/or associated DNA comprises a proteomics method.
5. The method according to embodiment 2, wherein the step of analysing the isolated nucleosomes and/or associated DNA comprises mass spectrometry.
6. A method for isolating purified circulating tumor DNA (ctDNA) from a biological sample, wherein said method comprises the steps of:
   (i) isolating circulating cell free nucleosomes containing histone H3.1 and/or H3.2 and/or H3t;
   (ii) extracting DNA from the nucleosome sample produced in step (i); and
   (iii) analysing the extracted DNA.
7. The method according to embodiment 6, wherein the step of analysing the extracted DNA comprises: DNA sequencing, methylated DNA sequencing analysis, PCR, BEAMing, NGS (targeted or whole genome), digital PCR, cold PCR (co-amplification at lower denaturation temperature-PCR), MAP (MIDI-Activated Pyrophosphorolysis), PARE (personalized analysis of rearranged ends) or Mass Spectrometry.
8. An immunoassay method for detecting an epigenetic epitope of tumor derived circulating nucleosomes in a biological sample, wherein said method comprises the steps of:
   (i) contacting the sample with a histone H3.1 and/or H3.2 and/or H3t binding agent;
   (ii) contacting the nucleosomes or sample with a second binding agent which binds to said epitope;
   (iii) detecting and/or quantifying the binding of said second binding agent to said epitope; and
   (iv) using the presence or degree of such binding as a measure of the presence of the particular epitope of tumor derived nucleosomes in the sample.
9. An immunoassay method for detecting an epigenetic epitope of tumor derived circulating nucleosomes in a biological sample wherein said method comprises the steps of:
   (i) contacting the sample with a first binding agent which binds to said epitope;
   (ii) contacting the nucleosomes or sample with a histone H3.1 and/or H3.2 and/or H3t binding agent;
   (iii) detecting and/or quantifying the binding of said histone H3.1 and/or H3.2 and/or H3t binding agent to nucleosomes in the sample; and
   (iv) using the presence or degree of such binding as a measure of the presence of the particular epitope of tumor derived nucleosomes in the sample.
10. The method according to embodiment 8 or embodiment 9, wherein the epitope comprises a histone modification.
11. The method according to embodiment 8 or embodiment 9, wherein the epitope comprises a modified nucleotide.
12. The method according to embodiment 8 or embodiment 9, wherein the epitope comprises a histone variant or isoform.
13. The method according to embodiment 8 or embodiment 9, wherein the epitope comprises a nucleosome adduct.
14. The use or method according to any one of embodiments 1 to 13, wherein the biological sample comprises a blood, serum or plasma sample.
15. A method of diagnosing cancer which comprises the step of detecting circulating cell free nucleosome associated histone variant H3.1 and/or H3.2 and/or H3t in a biological sample obtained from a human or animal subject.
16. The method according to embodiment 15, which additionally comprises detecting one or more histone modification, modified nucleotide, histone variant or isoform or nucleosome adduct.
17. The method according to embodiment 16, wherein the histone modification comprises H3K27Ac and/or 5-methylcytosine.
18. Use of a kit comprising a histone H3.1 and/or H3.2 and/or H3t binding agent in a method described in any one of embodiments 2 to 17.
19. The use or methods according to any one of embodiments 1 to 18, wherein the histone H3.1 and/or H3.2 and/or H3t binding agent comprises a H3.1 binding agent.

## Claims

1. An immunoassay method comprising the steps of:
(i) contacting a biological sample with a histone H3.1 and/or H3.2 and/or H3t binding agent;
(ii) contacting the biological sample with a second binding agent which binds to an epitope of circulating nucleosomes;
(iii) detecting and/or quantifying the binding of said second binding agent to nucleosomes in the biological sample; and
(iv) analysing the isolated nucleosomes and/or associated DNA.

2. The method according to claim 1, wherein the step of analysing the isolated nucleosomes and/or associated DNA comprises an immunoassay method.

3. The method according to claim 1, wherein the step of analysing the isolated nucleosomes and/or associated DNA comprises a proteomics method.

4. The method according to claim 1, wherein the step of analysing the isolated nucleosomes and/or associated DNA comprises mass spectrometry.

5. The method according to any one of claims 1 to 4, wherein the epitope comprises a histone modification.

6. The method according to any one of claims 1 to 4, wherein the epitope comprises a modified nucleotide.

7. The method according to any one of claims 1 to 4, wherein the epitope comprises a histone variant or isoform.

8. The method according to any one of claims 1 to 4, wherein the epitope comprises a nucleosome adduct.

9. The method according to any one of claims 1 to 8, wherein the biological sample comprises a blood, serum or plasma sample.

10. The method according to any one of claims 1 to 9, wherein the histone H3.1 and/or H3.2 and/or H3t binding agent is an antibody.

11. The method according to any one of claims 1 to 9, wherein the second binding agent is an antibody.
